# EUROPEAN PATENT APPLICATION

(11) **EP 1 216 708 A1**
(43) Date of publication of application: **26.06.2002**
(21) Application number: 01403329.4
(22) Date of filing: 20.12.2001
(51) Int. Cl.: A61K 38/47, A61L 2/00

(54) **A disinfectant for teats of animals, and a method for improving microorganism-environment harmful for animals**

(30) Priority: 21.12.2000 JP 2000388005
(71) Applicant: Shinei Fermentec Corporation, Tokyo (JP)
(72) Inventor: Ito, Naoki, c/o Shinei Fermentec Corporation, Tokyo (JP); Masayoshi, Iwahara, c/o Shinei Fermentec Corp., Tokyo (JP)
(74) Representative: Catherine, Alain

(57) **Abstract**

A novel disinfectant for teats of animals is provided. The disinfectant for teats of animals comprises lysozyme.

## Description

### Field of the invention

The present invention concerns to a harmful microorganism-removing agent from teats of animals, and amethod for improvingmicroorganism-environment harmful for animals.

### Prior art

The disinfection of teats of milking animals, in particular dairy cattle is a most important one of measures for prevention of mastitis. One disinfectant for teats has been developed in UK by Odd et al. in 1952, and has been since used in many countries. In Japan, such disinfectant has been used as a government project for improvement of milk quality since approximately 1975, which is at the present time employed in about 40% of Japanese total dairy cattle.

The method for disinfection of teats of milking cattle that is commonly carried out, includes dipping teats in medical fluid containing such disinfectant as described above to pasteurize or sterilize mastitis-causing bacteria deposited onto the surface of teat skin (namely, post-dipping), and employing wetting agent to improve the status of teat skin such as cracks, whereby inhibiting the growth of bacterium on the surface of teat and preventing mastitis. A number of such disinfecting preparations are currently sold in the market.

However, in the investigation accomplished by the National Institute for Research in Dairying in USA, it is reported that although new infection are decreased by 50% during the period of 12 months in milking cattle population which undergo post-dipping, such decrease of infection is only 14% in a group of cattle which have already infected, and thus subclinical infection by pre-existing mastitis-causing bacteria is likely to be sustained. Namely, although the post-dipping treatment decreases the rate of fresh infection, it is unable to be expected that preventive effects against mastitis-causing bacteria for what is called environmental mastitis is obtained.

The word "environmental mastitis" is meant by mastitis which is developed with occasion or the like in that mastitis-causing bacterium which has deposited onto teat skin and migrated to teat tip penetrates into teat cistern through teat orifice, based on counter flow phenomenon of milk which may occur during milking by milker. The duration of pasteurizative effect is relatively short (for example, 1-2 hours after dipping), and such effect disappears up to next milking, with pasteurization after milking i.e. post-dipping alone, and therefore, the effect for environmental mastitis-causing bacterium is limited.

As described above, in said post-dipping treatment, there are problems that it is unable to expect effect against infection from pre-existing bacterium and that effect against the environmental mastitis is also limited. Technology proposed in order to overcome such prior problems is a treatment for pasteurizing or sterilizing teats before milking (pre-dipping method). It was reported by Nakagawa, Furukawa and Nakajima that *Staphylococcus aureus* and *Streptococcus* were significantly less inhabited and milk quality was maintained more healthily in a farm in which pre-dipping was carried out, compared to one in which washing by towel was carried out, and thus pre-dipping treatment was thought to be effective.

As such, there is a literature disclosed that the dip pasteurization of teats before milking (hereafter, refers to pre-dipping) is effective for prevention from mastitis. However, since the pre-dipping is a treatment before milking, certain problem occurs that when the post-dipping agent is used as pre-dipping agent, it has high viscosity, iodine contained therein adheres to teats for any length of time, whereby iodine migrates into milk and retains in milk stocks . Therefore, it was shown that such agent adhered to teats after pre-dipping needs to be sufficiently removed.

Accordingly, there are needed a development of low or less iodine containing products which have low after-effectiveness (viscosity) . The post-dipping agent and pre-dipping agent should be products which meet the respective demand, respectively. However, there is yet obtained no suitable product which can be employed as pre-dipping agent in Japan.

Therefore, since development of mastitis is a serious problem for dairy farmers, it may be thought that there are problems such as use of the post-dipping agent as pre-dipping, use of the post-dipping agent departing from the indication of animal medicine, migration of iodine into milk and the like. In order to settle these problems, appearance of disinfectant for teats which can be effectively as pre-dipping agent, is desired.

### Summary of the Invention

The present inventors have found that by earnestly studying various substances having anti-bacteria property against mastitis-causing bacteria, in particular *Staphylococcus* and giving no adverse effect to human, lysozyme can possess such anti-bacteria property against *Staphylococcus aureus.*

The present invention is on the basis of said finding, and provides a novel disinfectant for teats of animals, and a method for improving microorganism- environment harmful for animals.

The above objects can be accomplished by any one of the following aspects (1)-(10):
(1) A disinfectant for teats of animals comprising lysozyme.
(2) The disinfectant according to the above (1) wherein said lysozyme is albumen lysozyme from fowls.
(3) The disinfectant according to the above (1) wherein said disinfectant is an aqueous solution containing lysozyme in amounts of 0.5 wt.% or more.
(4) The disinfectant according above (3) wherein said disinfectant is an aqueous solution containing lysozyme in amounts of 1.0 wt. % or more.
(5) The disinfectant according to the above (1) wherein said disinfectant further comprises glycine.
(6) A method for improving microorganism- environment harmful for animals comprising spraying lysozyme in barn of animals.
(7) The method according to the above (6) wherein said lysozyme is albumen lysozyme from fowls.
(8) The method according to the above(6) wherein said lysozyme is sprayed in form of an aqueous solution containing lysozyme in amounts of 0.5 wt. % or more.
(9) The method according to the above (8) wherein said lysozyme is sprayed in form of an aqueous solution containing lysozyme in amounts of 1.0 wt. % or more.
(10) A method for improving microorganism-environment harmful for animals comprising spraying lysozyme in surroundings where animals live.

### Detailed Description of the Invention

The disinfectant for teats of animals of the present invention is usually in form of aqueous solution and may contain lysozyme preferably in amounts of 0.5 wt. % or more, more preferably in amounts of 1 wt. % or more. The upper limit of lysozyme contents is not specifically given, but such upper amounts of about 10 wt. % is economically preferred. The disinfectant for teats of animals of the present invention may be powders of lysozyme as it is.

A preferable example of the lysozyme is albumen lysozyme from fowls, because it is cheaply and easily available. The lysozyme used in the present invention can lyze cell walls of *Staphylococcus aureus* to inhibit the growth of this bacterium.

The disinfectant for teats of animals according to the present invention may contain a suitable mount of glycine. This substance is thought to exhibit synergistic effect in combination with the lysozyme, as inhibitor to the cell wall of the bacteria. The glycine is preferably added in amounts of about 0.5-2 wt. % into the disinfectant of the present invention.

The disinfectant for teats of animals of the present invention may be applied to teats of animals by dipping (pre-dipping, post-dipping or both thereof), spraying, coating or the like.

From the results of experiment conducted by the present inventors, it is found that the disinfectant for teats of animals of the present invention can exhibit inhibitive action against *Bacirrus Subtilis* or the like other than *Staphylococcus aureus.* The disinfectant of the present invention can have also action of protective agent for diary cattle or other milking animals.

The lysozyme described above can be employed in improving microorganism- environment harmful for animals in barn of animals. In this case, the lysozyme may be merely sprayed, as it is or in form of aqueous solution, into surroundings where animals live. Thereby, the growth of *Staphylococcus aureu* can be suppressed to improve microorganism-environment harmful for animals.

The present invention will be further illustrated in the following examples. However, it is to be understood that these examples are for illustrative purposes only, and should not be used to limit the scope of the present invention in any manner.

### Examples

### Example 1

### Preparation of Bacterium Liquid

*Staphylococcus aureus* IFO 3060 was inoculated in bouillon medium (Bacto-pentone, 1 %; Beef extract, 0.5 %; Sodium chloride, 0.5 %), and cultured at 30°C for 18 hours by shaking to form a liquid containing said bacterium (about 10⁹/ml).

### Experimental Procedure

Into five of 30 ml of sample bottles, 5 ml of the same bouillon medium as above was placed, and after sterilizing, inoculated with 100 µl of the bacterium containing liquid. Thereafter, into the resulting medium, lysozyme was added in various amounts shown in Table 1 below to prepare Samples 1-5. After sealing, each sample bottle was set on a bio-thermo analyzer (Bio Thermo Analyzer Model H-201; Nippon Ikaki Seisakusho K.K. in Japan) and the exothermic value of each Sample was continuously determined and recorded. The exothermic value was as electric value (µ V) in order to keep Samples at 30°C. The results of determination were shown in Table 1 below. "bio-thermo analyzer" is meant by a device by which exothermic value developed as bacteria propagate is timely determined.

In Sample 1 and 2 which contain, respectively, 0 and 0.25 wt. % of lisozyme, *Staphylococcus aureus* began to grow after about 5 hours. As seen from Table 1, however, in Sample 3 containing 0.5 wt. % of lysozyme, there was observed suppressive effect to the bacterium in a time period of about 12 hours. Further, in Sample 4 and 5 containing 1 wt. % or more of lysozyme, the growth of the bacterium was not observed even after 12 days. From these results, it is apparent that lysozyme exhibits inhibitive effect against *Staphylococcus aureus*. In addition, since the above experiments were conducted at such conditions in which *Staphylococcus aureus* can most easily grow, better result may certainly be obtained at more severe conditions such as by lowering the temperature.

Further, when 1 wt. % of gycine was added into Sample 3, and the length of time at which the bacterium could be inhibited was determined, such length of time was longer than in Sample 3 containing no glycine.

### Example 2

When an aqueous solution containing 1 wt. % of lysozyme was prepared and used as dipping agent to carrying out post-dipping of teats of cattle by using a conventional method, the number of *Staphylococcus aureus* on the skin surface of teats were decreased.

### Example 3

Furthermore, the aqueous solution prepared in Example 2 above was sprayed into barn of cattle. In this ambient condition, the number of *Staphylococcus aureus* on the ground of floor were apparently degreased, as determined by a conventional method.

As described above, the effects obtained from the present invention is clear.

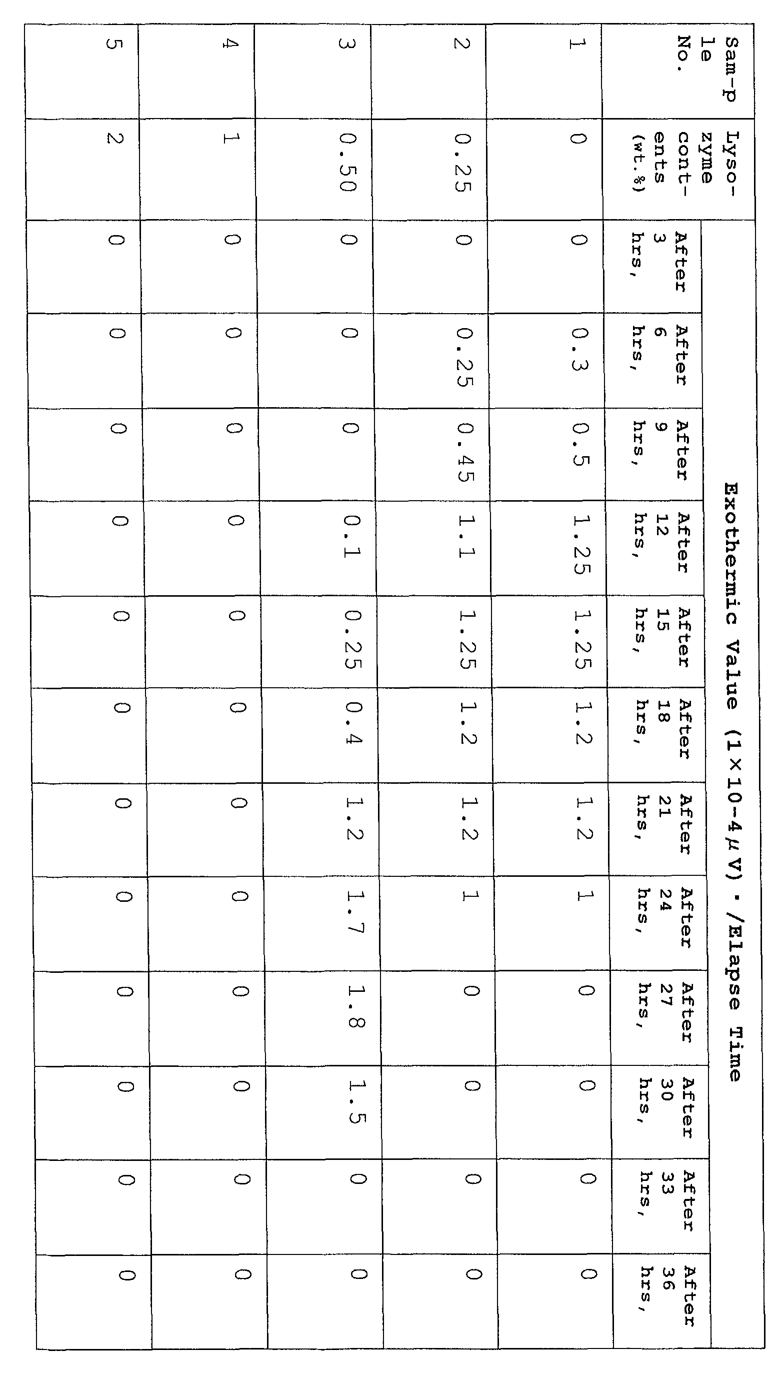

## Claims

1. A disinfectant for teats of animals comprising lysozyme.

2. The disinfectant according to Claim 1 wherein said lysozyme is albumen lysozyme from fowls.

3. The disinfectant according to Claim 1 wherein said disinfectant is an aqueous solution containing lysozyme in amounts of 0.5 wt.% or more.

4. The disinfectant according claim 3 wherein said disinfectant is an aqueous solution containing lysozyme in amounts of 1.0 wt. % or more.

5. The disinfectant according to Claim 1 wherein said disinfectant further comprises glycine.

6. A method for improving microorganism- environment harmful for animals comprising spraying lysozyme in barn of animals.

7. The method according to Claim 6 wherein said lysozyme is albumen lysozyme from fowls.

8. The method according to Claim 6 wherein said lysozyme is sprayed in form of an aqueous solution containing lysozyme in amounts of 0.5 wt. % or more.

9. The method according to Claim 8 wherein said lysozyme is sprayed in form of an aqueous solution containing lysozyme in amounts of 1.0 wt. % or more.

10. A method for improving microorganism-environment harmful for animals comprising spraying lysozyme in surroundings where animals live.
